# EUROPÄISCHE PATENTSCHRIFT

(11) **EP 0 779 795 B1**
(45) Veröffentlichungstag und Bekanntmachung des Hinweises auf die Patenterteilung: **17.06.1998**
(21) Anmeldenummer: 95928929.9
(22) Anmeldetag: 04.09.1995
(51) Int. Cl.: A61B 17/60

(54) **VORRICHTUNG ZUR EXTERNEN FIXIERUNG VON FRAKTUREN**
DEVICE FOR EXTERNALLY SECURING FRACTURES
DISPOSITIF D'OSTEOSYNTHESE EXTERNE

(30) Priorität: 07.09.1994 CH 2737/94
(43) Veröffentlichungstag der Anmeldung: 25.06.1997
(73) Patentinhaber: Weigum, Hans, 4435 Niederdorf (CH)
(72) Erfinder: WEIGUM, Hans, CH-4435 Niederdorf (CH); ILLI, Oscar, E., CH-8603 Schwerzenbach (CH)
(74) Vertreter: Zimmermann, Hans, Dr.
(86) Internationale Anmeldenummer: CH9500189
(87) Internationale Veröffentlichungsnummer: WO9607362

(56) Entgegenhaltungen:
- EP-A- 0 517 939
- EP-A- 0 524 441
- WO-A-88/01488
- DE-U- 9 005 149

## Beschreibung

Die vorliegende Erfindung betrifft eine Vorrichtung zur externen Fixierung von Fraktuzen, insbesondere der Extremitäten und des Beckens, bestehend mindestens aus einem Zug-/Druckstab sowie mehreren Knochenschrauben (Schanz'sche Schrauben) oder Knochennägeln (Steinmann-Nägel) und Verbindungsklemmen zur Herstellung der Verbindung zwischen den Knochenschrauben und dem kraftübertragenden Zug-/Druckstab, gemäss dem Oberbegriff des Patentanspruches 1.

Mit dieser Vorrichtung, die in der Fachsprache meist als Fixateur extern bezeichnet wird, werden auf operativem Wege insbesondere offene, infizierte Frakturen sowie Trümmerbrüche behandelt. Im Gegensatz zur internen Fixation mittels Implantaten ist es bei der Verwendung der genannten Vorrichtung nicht erforderlich, den Knochen im Frakturbereich freizulegen. Die erheblich vereinfachte Anwendung erlaubt den Einsatz einer solchen Vorrichtung auch durch paramedizinisches Personal, wie beispielsweise Nothelfer und Militärsanitäter. Das Haupteinsatzgebiet der Vorrichtung zur externen Fixierung von Frakturen ist im wesentlichen in der Kriegs- und Katastrophenmedizin sowie der Unfallchirurchie als rasche, provisorische oder definitive Versorgung zu sehen. Die Belastbarkeit eines derart fixierten Knochens ist sogleich nach der operativen Anbringung so gross, dass ein Patient danach sogleich für die spitalexterne Behandlung entlassen werden kann. Dies ist in vielen medizinisch unterversorgten Ländern mit einem akuten Mangel an Spitalbetten ein wirtschaftlich wesentlicher Faktor. Es ist daher offensichtlich, dass die wirtschaftlichen Ueberlegungen beim Kauf solcher Vorrichtungen von eminenter Bedeutung sind.

Aus der EP-A-0 524 441 ist eine Vorrichtung zur externen Fixierung von Frakturen gemäss dem Oberbegriff des Patentanspruches 1 bekannt, die Verbindungsklemmen umfasst, die einen zylindrischen Trägerkörper umfasst, mit zwei axial versetzten Durchgangslöchern in der Form von exzentrischen Ausnehmungen im Trägerkörper. Diese Ausnehmungen sind entsprechend dem Durchmesser der Knochenschrauben beziehungsweise des Zug-/Druckstabes dimensioniert. Der Zug-/Druckstab beziehungsweise die Knochenschraube werden im Trägerkörper durch Ueberwurfhülsen gehalten, auf die eine entsprechende Mutter mittelbeziehungsweise unmittelbar drückt.

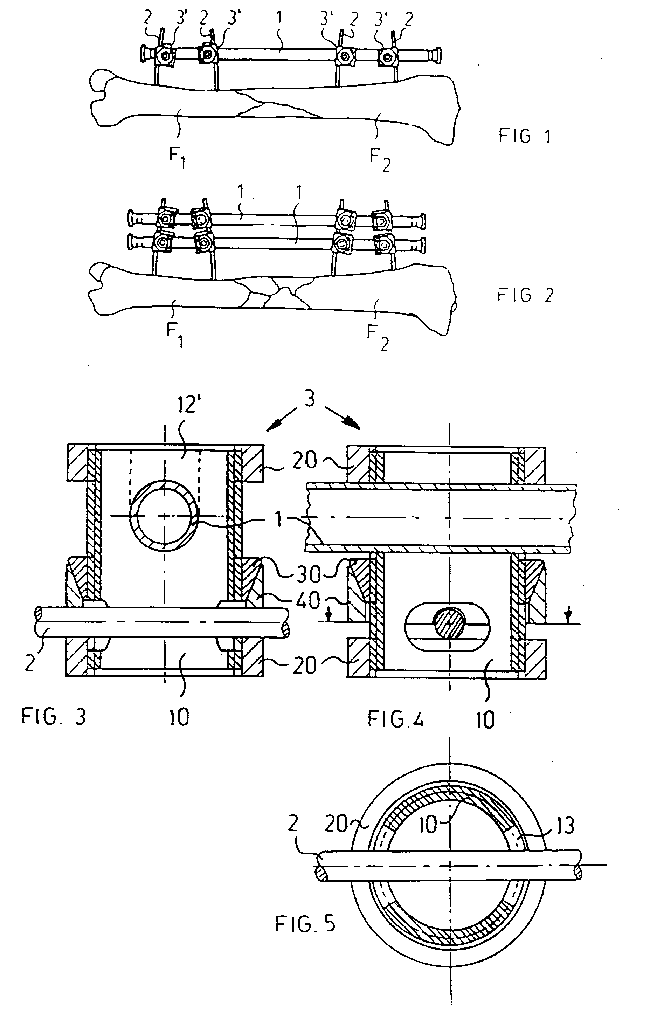

Betrachtet man die neuesten Entwicklungen auf diesem Gebiet, so stellt man fest, dass die meisten Neuerungen in Richtung einer Perfektion mit immer genaueren Anpassmöglichkeiten und kostspieligem Zubehör führt. Kostenmässig eines der teuersten Teile der gesamten Vorrichtung stellt die Verbindungsklemme dar, welche zur Herstellung der Verbindung zwischen einer Knochenschraube und dem kraftübertragenden Zug-/Druckstab dient. Eine weitverbreitete Verbindungsklemme ist aus der Darstellung gemäss Figur 1 mindestens andeutungsweise ersichtlich. Eine solche Klemme besteht aus einem U-förmigen Halteteil, mit einer beide Wangen durchsetzenden Bohrung und einem zwischen den beiden Wangen gelagerten Klotz mit einer gleich dimensionierten Durchgangsbohrung. Durch die Durchgangsbohrung wird der Zug-/Druckstab durchgeführt und die Klemmung erfolgt, indem der Klotz mittels einer Schraube relativ zum U-förmigen Körper verschoben werden kann. Des weiteren umfasst die Klemme ein Klemmbackenpaar, das mit einer weiteren Schraube zusammengepresst wird. Ferner ist die relative Winkellage der beiden Backen zueinander gesichert und es sind Mittel vorgesehen, um die relative Winkellage der Klemmbacken auf dem Körper der Klemme zu fixieren. Sämtliche Teile der Klemme stellen hohe Forderungen an die Fertigung, müssen äusserst präzis gearbeitet sein und sind entsprechend auch teuer und entsprechend empfindlich gegen Ueberbelastung und Beschädigung.

Es ist daher die Aufgabe der vorliegenden Erfindung, eine Vorrichtung der eingangs genannten Art zu schaffen mit einer Verbindungsklemme, die erheblich preiswerter hergestellt werden kann, weniger Präzision bedarf und trotzdem ein hohes Mass an Anpassbarkeit bezüglich der Winkellage der verschiedenen zueinander verlaufenden und zu verbindenden Teile gewährt.

Diese Aufgabe löst eine Vorrichtung der eingangs genannten Art mit den Merkmalen des kennzeichnenden Teiles des Patentanspruches 1.

Bei der Fertigung kleinerer Serien oder bei der Fertigung mit möglichst einfachen Werkzeugen kann vorteilhafterweise die Verbindungsklemme aus einem Trägerkörper bestehen, der aus einem Abschnitt eines Gewinderohres gefertigt sein kann. Auf dieser Basis lassen sich dann alle weiteren Teile der Verbindungsklemme als einfache Drehteile fertigen. Gestaltet man den rohrförmigen Trägerkörper der Verbindungsklemme der erfindungsgemässen Vorrichtung entsprechend den Merkmalen des Patentanspruches 7, so lässt sich dieser auch als Gussteil fertigen, was eine besonders preiswerte Herstellung ergibt, falls eine entsprechend grosse Stückzahl gefertigt wird.

Aus den abhängigen Patentansprüchen gehen weitere vorteilhafte Ausgestaltungsformen des Erfindungsgegenstandes hervor, die in der Zeichnung dargestellt und in der nachfolgenden Beschreibung erläutert sind.

In der Zeichnung sind zwei bevorzugte Ausführungsbeispiele des Erfindungsgegenstandes dargestellt. Es zeigt:
- Figur 1: - zeigt eine Vorrichtung zur externen Fixierung von Frakturen mit herkömmlichen verbindungsklemmen gemäss Stand der Technik und
- Figur 2: - dieselbe Vorrichtung in einer etwas komplexeren Anwendung.
- Figur 3: - zeigt eine bevorzugte Ausführungsform der erfindungsgemässen Verbindungsklemme im axialen Längsschnitt parallel zur Verlaufsrichtung der Knochenschraube und
- Figur 4: - einen Schnitt in der Verlaufsrichtung des Zug-/Druckstabes, während die
- Figur 5: - einen Schnitt senkrecht zur Längsachse der Verbindungsklemme in der Durchstossebene der Knochenschraube darstellt. Die
- Fig. 6-8: - zeigen zwei Längs- und einen Querschnitt durch einen Trägerkörper einer ersten Ausführungsform der Verbindungsklemme und
- Fig. 9-11: - zwei Längsschnitte und eine Aufsicht auf einen Trägerkörper einer zweiten Ausführungsform der Verbindungsklemme.
- Figur 12: - zeigt einen Querschnitt durch einen Klemmring, der in
- Figur 13: - in der Aufsicht dargestellt ist.
- Figur 14: - zeigt einen Axialschnitt durch einen Stützring und
- Figur 15: - zeigt denselben in der Aufsicht, während
- Figur 16: - einen Klemmring im Schnitt und
- Figur 17: - in der Ansicht von unten darstellt.
- Figur 18: - zeigt eine Verbindungsklemme mit innen liegenden Klemmringen.

Auf die rein operative Anwendung der Vorrichtung zur externen Fixierung von Frakturen wird hier nicht eingegangen. Hierzu kann beispielsweise auf den Artikel von F. Behrens und K. Searls mit dem Titel "External Fixation of the Tibia" in British Journal of Bone and Joint Surgery, 68-93 (1986), Seiten 246-254 verwiesen werden.

Die Verbindung zwischen Knochenfragmenten und Fixateur erfolgt generell entweder mit Knochenschrauben oder Knochennägeln. Der Einfachheit halber wird hier jedoch nur von Knochenschrauben gesprochen, die häufiger zum Einsatz kommen als Knochennägel. Bei den Knochenschrauben verwendet man häufig Schanz'sche Schrauben.

In den Figuren 1 und 2 erkennt man deutlich, wie zwei Knochenfragmente F1 und F2 mittels einer bekannten Vorrichtung zueinander stabilisiert sind. Der Zug-/Druckstab 1 verläuft in etwa parallel zur Verlaufrichtung des zu fixierenden Knochens, während die Knochenschrauben 2 etwa senkrecht zu der Verlaufrichtung des Knochens in denselben eingeschraubt sind. Die Verbindungsklemmen 3 sind einerseits vom Zug-/Druckstab 1 durchsetzt und halten andererseits klemmend die Knochenschrauben 2. Dabei sind in jedem der beiden Hauptknochenfragmente jeweils zwei Knochenschrauben eingesetzt. Dies ist für die exakte Lagefixierung und Achsenstabilisierung relativ zum Zug-/Druckstab 1 erforderlich. Verschiedene Bedingungen mögen es notwendig machen, dass für die Fixation einer Fraktur mehr als ein Zug-/Druckstab erforderlich ist. Dabei können beispielsweise zwei oder auch drei Zug-/Druckstäbe 1 an denselben Knochenschrauben 2 befestigt sein.

Sie können aber auch mittels unterschiedlicher Knochenschrauben mit den Knochenfragmenten verbunden sein. In solchen Fällen können auch Verbindungsstäbe zwischen den Zug-/Druckstäben angebracht sein, die wiederum mittels den Verbindungsklemmen gehalten werden.

In der Zeichnung sind die den Stand der Technik darstellenden Verbindungsklemmen in den Figuren 1 und 2 mit 3' bezeichnet, während die neuen, erfindungsgemässen Verbindungsklemmen in ihrer Gesamtheit, wie sie die Figuren 3-5 zeigen, mit 3 bezeichnet sind.

Wie aus den Figuren 1 und 2 deutlich ersichtlich, müssen folglich die Verbindungsklemmen 3 einerseits die Zug-/Druckstäbe 1 und andererseits die Knochenschrauben 2 fixierend halten können. Die erfindungsgemässe Verbindungsklemme 3 besteht hierzu aus einem Trägerkörper 10 und verschiedenen auf dem Trägerkörper 10 angeordneten Ringen. Der Trägerkörper 10 weist zwei in axialer Richtung zueinander versetzte Durchgangslöcher 12 und 13 auf. Das diametral verlaufende Durchgangsloch 12 ist eine zylindrische Bohrung, die so dimensioniert ist, dass der kraftübertragende Zug-/Druckstab 1 die Bohrung mit relativ geringem Spiel durchsetzen kann. Die Bewegungsfreiheit des Trägerkörpers 10 relativ zum Zug-/Druckstab 1 beschränkt sich folglich auf eine Verschiebung des Trägerkörpers 10 in Richtung der Längsachse des Zug-/Druckstabes 1 sowie auf eine Rotation um die genannte Längsachse des Zug-/Druckstabes 1.

Weil der Stab 1 in der Verwendung sowohl Druck- als auch Zugbelastungen überträgt, wird er generell als Zug-/Druckstab bezeichnet.

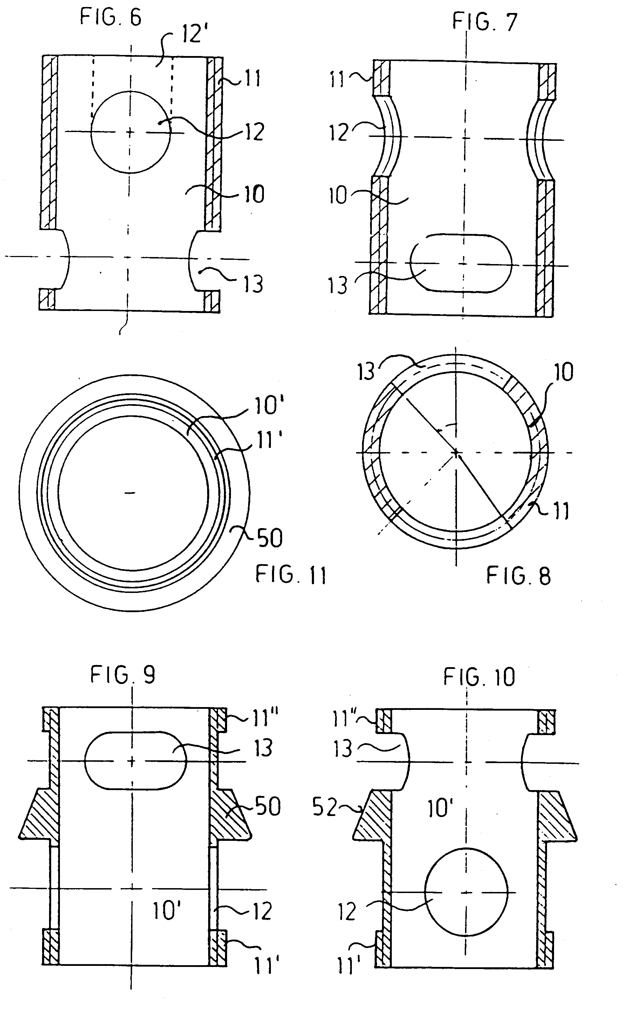

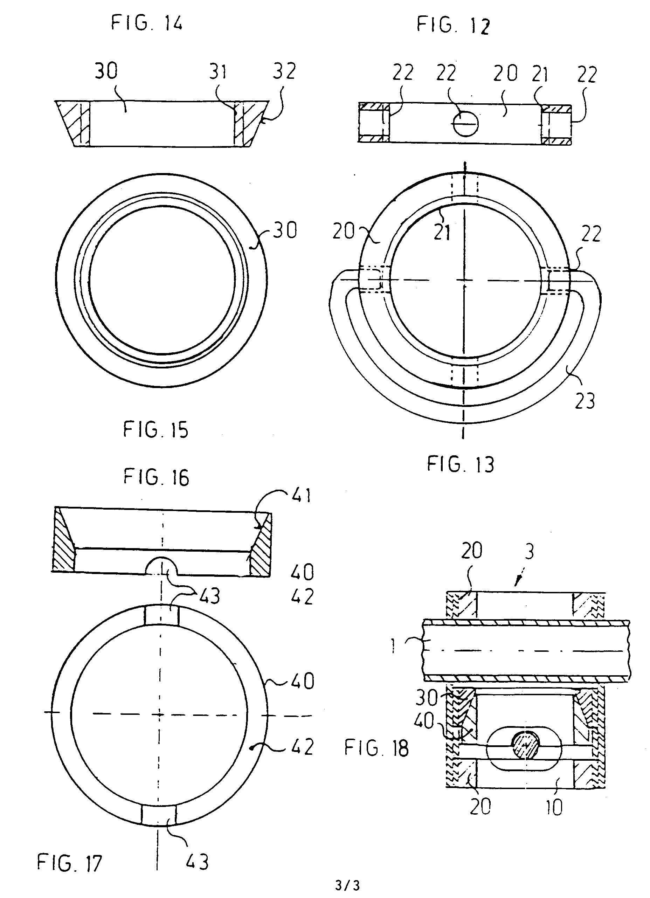

Wie aus den Figuren 6-8 ersichtlich, kann der Trägerkörper 10 aus einem Gewinderohrabschnitt gefertigt sein. In diesem Falle weist der rohrförmige Trägerkörper 10 ein über die gesamte Länge verlaufendes Aussengewinde 11 auf. Die Fixierung des Zug-/Druckstabes 1 im Trägerkörper 10 lässt sich auf einfachste Weise dadurch realisieren, indem man auf dieses Aussengewinde 11 einen Klemmring 20 mit Innengewinde 21 aufschraubt. Ein solcher Klemmring weist Angriffsflächen für ein Schraubwerkzeug auf. Im dargestellten Beispiel gemäss den Figuren 12 und 13 sind diese Angriffsflächen in der Form von radialen Bohrungen 22 gestaltet. Ein Klemmring, der als Angriffsflächen Bohrungen aufweist, ist beispielsweise geeignet, um mittels einem Hakenschlüssel angezogen zu werden. In einer besonderen Ausführungsform können auch zwei einander diametral gegenüberliegende Bohrungen zur Aufnahme eines lösbaren Bügels dienen. Dieser Bügel, der schwenkbar mit dem Klemmring 20 verbunden ist, erlaubt die schnelle und werkzeugfreie Befestigung des Klemmringes auf dem Trägerkörper 10 und mit ihm lässt sich genügend Kraft übertragen, um eine Klemmung zu erreichen, die für eine provisorische Lagefixierung genügt. Für die abschliessende definitive Fixierung kann entweder ein rohrförmiger Schlüssel über den Bügel geschoben werden , der diesen für die Momentübertragung auf den Klemmring benutzt, oder der Bügel 23 wird entfernt und der Klemmring wird mit dem Hakenschlüssel festgezogen. Selbstverständlich kann der Klemmring 20 auch mit planen Angriffsflächen auf der Aussenfläche ausgerüstet sein, so dass der Klemmring ähnlich einer Mutter beispielsweise mittels einem Gabelschlüssel angezogen werden kann. Selbstverständlich sind auch andere Ausgestaltungsformen, die entsprechenden, bekannten Werkzeugen angepasst sind, realisierbar.

Ein zweites diametral den Trägerkörper 10 durchsetzendes Durchgangsloch 13 dient der Durchführung einer Knochenschraube. Während der Zug-/Druckstab 1 im Trägerkörper 10 formschlüssig gehalten ist, trifft dies auf die Durchführung der Knochenschraube durch den Trägerkörper 10 nicht zu. Vielmehr muss die Knochenschraube 2 genügend Bewegungsfreiheit haben, um die erforderliche Winkelanpassung vornehmen zu können. Entsprechend ist das Durchgangsloch 13 für die Knochenschraube eher wie ein Fenster gestaltet. In der vorliegenden Ausgestaltungsform ist das Durchgangsloch 13 als Langloch realisiert. Die Klemmung der Knochenschraube erfolgt hier nicht zwischen einem zweiten Klemmring 20 und dem Trägerkörper 10, sondern zwischen einem zweiten Klemmring 20 und einem Anpassring 40.

Im Bereich zwischen den beiden Durchgangslöchern 12 und 13, die im Trägerkörper 10 axial versetzt und windschief senkrecht zueinander verlaufen, ist ein Stützring 30 auf das bereits erwähnte Aussengewinde 11 aufgeschraubt. Der Stützring 30 hat ein Innengewinde 31, welches auf das Aussengewinde 11 des Trägerkörpers 10 passt und nach der Montage permanent gegen Verdrehen gesichert ist. Die radiale Aussenfläche des Stützringes 30 ist vorzugsweise konisch gestaltet. Auf dem Stützring 30 liegt auf dessen konischer Aussenfläche ein Anpassring 40 auf, der eine entsprechend geneigte konische Innenfläche 41 hat. Der Anpassring 40 stellt eine bedingt bewegliche Klemmbacke dar. Zwischen dieser Klemmbacke und einem zweiten Klemmring 20 wird die Knochenschraube 2 gehalten. Vorteilhafterweise gestaltet man diese Halterung so, dass die Knochenschraube nicht nur kraftschlüssig, sondern auch formschlüssig gehalten ist. Entsprechend weist der Anpassring 40 an seiner planen Stirnfläche 42 paarweise offene einander diametral gegenüberliegende Ausnehmungen 43 auf. Die Tiefen der diversen über den Umfang verteilten Ausnehmungen 43 können selbstverständlich unterschiedlich sein, entsprechend den Durchmessern der zu klemmenden Knochenschrauben. Der Durchmesser üblicher Knochenschrauben variiert zwischen 3 und 5 Millimeter. Die Form der offenen Ausnehmungen 43 kann halbrund, halboval oder die Gestalt einer dreieckigen Kerbe aufweisen, um die Klemmwirkung zu steigern.

Während die Ausführungsform gemäss den Zusammenstellungszeichnungen der Figuren 3-5 aus den Einzelelementen gemäss den Figuren 6-8 sowie 12-17 besteht, zeigen die Figuren 9-11 eine Variante eines Trägerkörpers 10', der nicht aus einem Gewinderohr gefertigt ist, sondern beispielsweise aus einem dickwandigen Rohr gedreht oder vorteilhafterweise sogar als Gusselement gefertigt sein kann. Der Trägerkörper 10' hat einen Kragen 50, der einstückig mit dem Trägerköiper 10' verbunden ist und im Aussehen und in der Funktion dem Stützring 30 entspricht. Auch der Kragen 50 hat eine entsprechend konisch verlaufende radiale Aussenfläche 52. Diese dient wiederum als Auflagefläche für die konische Innenfläche 41 des Anpassringes 40. Herstellungsmässig bedingt verläuft nunmehr das Aussengewinde auf dem Trägerkörper 10 nicht mehr über die gesamte Länge desselben, sondern ist lediglich wo funktionsmässig nötig an den beiden Endbereichen angebracht. Diese Gewindeabschnitte sind entsprechend mit 11' und 11'' gekennzeichnet.

Im Prinzip kann die Klemmung zwischen dem Klemmring 20 und dem Anpassring 40 auch ohne die konische Gestaltung der zueinander gerichteten Anpressflächen von Stützring und Anpassring realisiert werden. Dies würde jedoch einen erhöhten Fertigungsaufwand erfordern und eine höhere Anzugskraft der zu verschraubenden Teile bedingen. Beides ist jedoch eher unerwünscht, so dass die Ausführungsform mit konischen Anpressflächen bevorzugt wird. Die Konizität der Anpressflächen ist eine Frage der Optimierung.

Es ist der grosse Vorteil der erfindungsgemässen Vorrichtung, dass diese in Bezug auf Werkstoffestigkeit und Fertigungsgenauigkeit erheblich geringere Ansprüche stellt, ohne dass Abstriche an der Qualität der Fixierung der Frakturteile zueinander gemacht werden müssen. Auch gegenüber im Einsatz vorkommenden Beschädigungen und Ueberlastungen ist die erfindungsgemässe Lösung erheblich toleranter als bekannte Systeme.

Die Konstruktion lässt auch eine Vielzahl von Varianten zu. So kann der Trägerkörper 10 statt der diametralen, zylindrischen Bohrung 12 mit einem offenen Schlitzpaar 12' versehen sein, wie dies in den Figuren 3 und 6 alternativ strichliniert eingezeichnet ist. Hierdurch kann die gesamte Verbindungsklemme 3 statt auf den Zug-/Druckstab aufgeschoben zu werden auch seitlich an den Stab angesetzt werden. Dies ist aus operativen Gründen gelegentlich erwünscht, wenn es sich zeigt, dass zur Fragmentstabilisierung eine weitere Knochenschraube erforderlich ist.

Während in den bisher beschriebenen Ausführungen der Trägerkörper 10 jeweils mit Aussengewinden versehen war und der konische Stützring aussen aufgesetzt war, kann dies selbstverständlich auch umgekehrt gestaltet werden, indem das Gewinde innen angeordnet ist und der Stützring ebenfalls nach innen ragt. Die Klemmringe 20 haben dann ein Aussengewinde und lassen sich zapfenartig in den Trägerkörper einschrauben. Die Klemmringe 20 müssen dann mit einem Innenantrieb, wie Innensechskant- oder -vierkant oder einem Torxantrieb versehen sein. Diese Variante erlaubt eine besonders platzsparende Lösung. Diese Variante ist systematisch in der Figur 18 dargestellt. Andeutungsweise haben die Klemmringe 20 einen Innensechskant.

## Patentansprüche

1. Vorrichtung zur externen Fixierung von Frakturen, insbesondere der Extremitäten und des Beckens, bestehend mindestens aus einem Zug-/Druckstab (1) sowie mehreren Knochenschrauben (2) sowie Verbindungsklemmen (3) zur Herstellung der Verbindung zwischen den Knochenschrauben und dem kraftübertragenden Zug-/Druckstab, wobei jede Verbindungsklemme (3) aus einem mindestens mit einem Gewinde (11,11',11'') versehenen Trägerkörper (10,10') mit mindestens zwei axial versetzten, den Trägerkörper diametral durchsetzenden Durchgangslöchern (12,13) besteht, deren Weite dem sie durchsetzenden Zug-/Druckstab (1) beziehungsweise der sie durchsetzenden Knochenschraube (2) angepasst sind, dadurch gekennzeichnet, dass der Trägerkörper rohrförmig ist und mit mindestens zwei auf das Gewinde am Trägerkörper passenden schraubbaren Klemmringen (20) versehen ist, zur jeweils klemmenden Fixierung des Zug-/Druckstabes (1) und der Knochenschraube (2) mit dem Trägerkörper (10,10') der Verbindungsklemme (3).

2. Vorrichtung nach Anspruch 1, dadurch gekennzeichnet, dass der Trägerkörper (10) aus einem Abschnitt eines Gewinderohres gefertigt ist.

3. Vorrichtung nach Anspruch 2, dadurch gekennzeichnet, dass zwischen den beiden axial versetzten, diametralen Durchgangslöchern (12,13) ein Stützring (30) befestigt ist, auf dem ein Anpassring (40) aufliegt, welcher als Klemmbacke einer Knochenschraube (2) dient, während eine zweite Klemmbacke durch einen der beiden schraubbaren Klemmringe (20) gebildet ist.

4. Vorrichtung nach Anspruch 3, dadurch gekennzeichnet, dass der Stützring (30) und der Anpassring (40) zueinander gerichtete Anpressflächen (32,41) aufweisen.

5. Vorrichtung nach Anspruch 3, dadurch gekennzeichnet, dass der Anpassring (40) in der dem Stützring abgelegenen Stirnfläche mehrere offene Ausnehmungen (43) aufweist zur formschlüssigen Lagerung einer Knochenschraube (2).

6. Vorrichtung nach Anspruch 5, dadurch gekennzeichnet, dass unterschiedliche Ausnehmungen (43) für unterschiedliche Durchmesser von Knochenschrauben angebracht sind.

7. Vorrichtung nach Anspruch 1, dadurch gekennzeichnet, dass der rohrförmige Trägerkörper (10') lediglich an den beiden Enden ein Aussengewinde (11',11'') aufweist und zwischen den beiden axial versetzten, diametralen Durchgangslöchern (12,13) ein Kragen (50) angeordnet ist, der eine Auflage für einen Anpassring (40) darstellt, welcher als Klemmbacke einer Knochenschraube (2) dient, während eine zweite Klemmbacke durch einen der beiden schraubbaren Klemmringe (20) gebildet ist.

8. Vorrichtung nach Anspruch 7, dadurch gekennzeichnet, dass der eine Auflage bildende Kragen (50) und der aufliegende Anpassring (40) zueinander gerichtete konische Anpressflächen (41,52) aufweisen.

9. Vorrichtung nach Anspruch 1, dadurch gekennzeichnet, dass die beiden schraubbaren Klemmringe (20) Angriffsflächen für ein Schraubwerkzeug aufweisen.

10. Vorrichtung nach Anspruch 9, dadurch gekennzeichnet, dass die Angriffsflächen Bohrungen (22) sind, die als Angriffsflächen für einen Hakenschlüssel dienen.

11. Vorrichtung nach Anspruch 10, dadurch gekennzeichnet, dass mindestens zwei einander annähernd diametral gegenüberliegende Bohrungen (22) vorgesehen sind und ein lösbarer Bügel (23) als Schraubhilfe darin gehalten ist.

12. Vorrichtung nach Anspruch 9, dadurch gekennzeichnet, dass die Angriffsflächen plane Flächen sind, die als Angriffsflächen für einen Gabelschlüssel dienen.

13. Vorrichtung nach Anspruch 9, dadurch gekennzeichnet, dass die beiden Klemmringe ein Aussengewinde aufweisen und die Angriffsflächen für ein Schraubwerkzeug ein Innenmehrkant bilden als Schraubhilfe für einen Steckschlüssel.

## Claims

1. A device for the external fixation of fractures, in particular of the extremities and the pelvis, consisting at least of a traction/compression rod (1) as well as several bone screws (2) as well as connecting clamps (3) for making the connection between the bone screws and the force-transmitting traction/compression rod, wherein each connecting clamp (3) consists of a support body (10, 10') provided with at least one thread (11, 11', 11''') and with at least two axially offset through-openings (12, 13) passing through the support body, whose widths are adapted to the traction/compression rod (1) passing through them or to the bone screw (2) passing through them, characterized in that the support body is pipe-shaped and provided with at least two clamping rings (20), which can be screwed on the thread on the support body, for the respective clamping fixation of the traction/compression rod (1) and the bone screw (2) with the support body (10, 10') of the connecting clamp (3).

2. The device in accordance with claim 1, characterized in that the support body has been made from a section of a threaded pipe.

3. The device in accordance with claim 2, characterized in that a support ring (30) is fastened between the two axially offset, diametrical through-bores (12, 13), on which an adapter ring (40) rests, which is used as a clamping cheek for a bone screw (2), while a second clamping cheek is constituted by one of the two screwable clamping rings (20).

4. The device in accordance with claim 3, characterized in that the support ring (30) and the adapter ring (40) have contact surfaces (32, 41), which are oriented toward each other.

5. The device in accordance with claim 3, characterized in that in the front face remote from the support ring, the adapter ring (40) has several open recesses (43) for the interlocking seating of a bone screw (2).

6. The device in accordance with claim 5, characterized in that different recesses (43) for different diameters of bone screws are provided.

7. The device in accordance with claim 1, characterized in that the pipe-shaped support body (10') has an exterior thread (11', 11'') only at its two ends, and a collar (50) is disposed between the two axially offset, diametrical through-openings (12, 13), which represents a support for an adapter ring (40), which is used as a clamping cheek for a bone screw (2), while a second clamping cheek is constituted by one of the two screwable clamping rings (20).

8. The device in accordance with claim 7, characterized in that the collar (50) constituting a support and the supported adapter ring (40) have conical contact surfaces (41, 52), which are oriented toward each other.

9. The device in accordance with claim 1, characterized in that the two screwable clamping rings (20) have engagement surfaces for a screw tool.

10. The device in accordance with claim 9, characterized in that the engagement surfaces are bores (22), which are used as engagement surfaces for a hook wrench.

11. The device in accordance with claim 10, characterized in that at least two bores (22), which are located approximately diametrically opposite each other, are provided and a releasable hoop (23) is held in them as a screw aid.

12. The device in accordance with claim 9, characterized in that the engagement surfaces are flat surfaces which are used as engagement surfaces for an open-end wrench.

13. The device in accordance with claim 9, characterized in that the two clamping rings have an exterior thread and the engagement surfaces for a screw tool form an interior polygon socket as a screw aid for a socket wrench.

## Revendications

1. Dispositif pour la fixation externe de fractures, notamment des extrémités et du bassin, constitué par au moins une tige travaillant en traction/compression (1) ainsi que plusieurs vis à os (2) ainsi que des pinces de liaison (3) servant à réaliser la liaison entre les vis à os et la tige de transmission de force travaillant en traction/compression, chaque pince de liaison (3) étant constituée par un corps de support (10, 10') pourvu d'au moins un filetage (11, 11', 11'') et comportant au moins deux trous traversants (12, 13), qui sont décalés axialement et traversent diamétralement le corps de support et dont les diamètres intérieurs sont adaptés à la tige travaillant en traction/compression (1) qui passe dans ces trous ou à la vis à os (2) qui passe dans ces trous, caractérisé en ce que le corps de support possède une forme tubulaire et comporte au moins deux bagues de serrage vissables (20), qui s'engagent dans le filetage situé sur le corps de support, pour la fixation par serrage de la tige travaillant en traction/compression (1) et de la vis à os (2) au corps de support (10, 10') de la pince de liaison (3).

2. Dispositif selon la revendication 1, caractérisé en ce que le corps de support (10) est constitué par un tronçon de tube fileté.

3. Dispositif selon la revendication 2, caractérisé en ce qu'entre les deux trous diamétraux traversants (12, 13) décalés axialement est fixée une bague d'appui (30), sur laquelle s'applique une bague d'adaptation (40), qui est utilisée comme mâchoire de serrage d'une vis à os (2), tandis qu'une seconde mâchoire de serrage est formée par l'une des deux bagues de serrage vissable (20).

4. Dispositif selon la revendication 3, caractérisé en ce que la bague d'appui (30) et la bague d'adaptation (40) possèdent des surfaces de serrage (32, 41), qui sont dirigées l'une vers l'autre.

5. Dispositif selon la revendication 3, caractérisé en ce que la bague d'adaptation (40) possède, dans la face frontale tournée à l'opposé de la bague d'appui, plusieurs évidements ouverts (43) servant à loger, selon une liaison par formes complémentaires, une vis à os (2).

6. Dispositif selon la revendication 5, caractérisé en ce que différents évidements (43) sont formés pour des vis à os de différents diamètres.

7. Dispositif selon la revendication 1, caractérisé en ce que le corps de support tubulaire (10') possède un filetage extérieur (11', 11'') uniquement au niveau de ses deux extrémités et qu'entre Tes deux trous traversants diamétraux (12, 13) décalés axialement est disposé un collet (50), qui représente un appui pour une bague d'adaptation (40), qui est utilisée en tant que mâchoire de serrage d'une vis à os (2), tandis qu'une seconde mâchoire de serrage est formée par l'une des deux bagues de serrage vissable (20).

8. Dispositif selon la revendication 7, caractérisé en ce que le collet (50) qui forme un appui, et la bague d'adaptation (40), qui est en appui sur ce collet, possèdent des surfaces de serrage coniques (41, 52) qui sont dirigées l'une vers l'autre.

9. Dispositif selon la revendication 1, caractérisé en ce que les deux bagues de serrage vissables (20) possèdent des surfaces d'attaque pour un outil de vissage.

10. Dispositif selon la revendication 9, caractérisé en ce que les surfaces d'attaque sont des perçages (22), qui sont utilisés comme surface d'attaque pour une clé à ergot.

11. Dispositif selon la revendication 10, caractérisé en ce qu'au moins deux perçages (22) approximativement diamétralement opposés sont prévus et qu'un étrier amovible (23) en tant que système auxiliaire de vissage est retenu dans ces perçages.

12. Dispositif selon la revendication 9, caractérisé en ce que Tes surfaces d'attaque sont des surfaces planes, qui sont utilisées comme surfaces d'attaque pour une clé à fourche.

13. Dispositif selon la revendication 9, caractérisé en ce que les deux bagues de serrage possèdent un filetage extérieur et que Tes surfaces d'attaque pour un outil de vissage forment un élément à plusieurs pans creux en tant que système auxiliaire de vissage pour une clé à douille.
